# EUROPEAN PATENT SPECIFICATION

(11) **EP 2 762 067 B1**
(45) Date of publication and mention of the grant of the patent: **28.09.2016**
(21) Application number: 14152078.3
(22) Date of filing: 22.01.2014
(51) Int. Cl.: A61B 5/0408, A61B 5/0478, A61B 5/0492, A61N 1/04

(54) **Sensor Platform and Method of Preparing the Same**
Sensorplattform und Verfahren zur Herstellung davon
Plate-forme de capteurs et procédé de préparation de celle-ci

(30) Priority: 04.02.2013 KR 20130012470
(43) Date of publication of application: 06.08.2014
(73) Proprietor: Samsung Electronics Co., Ltd, Gyeonggi-do 443-742 (KR)
(72) Inventor: Park, Sang Yun, 446-712 Gyeonggi-do (KR); Ko, Byung Hoon, 446-712 Gyeonggi-do (KR); Kim, Jong Pal, 446-712 Gyeonggi-do (KR)
(74) Representative: Grünecker Patent- und Rechtsanwälte PartG mbB

(56) References cited:
- EP-A1- 1 739 149
- US-A- 4 548 862
- US-A- 4 989 607
- US-A1- 2008 311 378
- US-A1- 2009 209 840
- US-A1- 2010 114 273
- US-A1- 2011 301 683
- US-B1- 6 327 487

## Description

### BACKGROUND

### 1. Field

The following description relate to a sensor platform and a method of preparing the same.

### 2. Description of Related Art

Bioelectrical signals, such as, for example, an electrocardiogram (ECG), an electromyogram (EMG), an electroencephalogram (EEG), an electrooculogram (EOG), and a galvanic skin response (GSR) signal, reflect health and fitness information of a subject. New techniques are being developed to measure, analyze, and apply a bioelectrical signal to disease management and health management. Due to a presence of electrolytes in a human body, all bioelectrical signals including ECG signals are transferred and carried by a flow of ions in the body, also known as an ionic current. Accordingly, measuring a bioelectrical signal using an external electronic device involves changing an ionic current to an electronic current. When an electrochemical reaction occurs during the change from an ionic current to an electronic current, as a charge exchange resistance at an electrode-electrolyte interface is lower, a potential drop across the interface may be reduced, a bioelectrical signal may be transmitted to an external measuring device with higher fidelity, and a signal to noise ratio (SNR) may be increased. An electrode having a low charge exchange resistance is called a non-polarizable electrode, and a common example is a silver-silver chloride electrode.

Different techniques are known in the art to produce highly conductive electrodes based on polymers. Document US 4 989 607 discloses a method of manufacturing a plurality of electrodes arranged on the same hydrogel sheet which includes a supporting net structure and an electrolyte. Document US 2010/114273 discloses an electrode formed on a hydrogel sheet doped with gold nano-particles.

Generally, two or more electrodes attached to a body surface are utilized for proper measurement of a bioelectrical signal, and a bioelectrical signal is measured by attaching a required number of electrodes to a body surface.

### SUMMARY

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter.

In one general aspect, there is provided a sensor platform including a hydrogel sheet comprising a net structure; an electrolyte applied to the net structure; and a plurality of electrodes disposed on the hydrogel sheet.

The hydrogel sheet may be flexible. This avoids inconvenience when attached to the human body.

The hydrogel sheet may have an anisotropic ionic conductivity. Even when a plurality of electrodes are disposed at arbitrary locations in a disordered arrangement on the hydrogel sheet, bioelectrical signals may be measured at a plurality of regions on a human body without causing an electrical interference between the electrodes. Unlike a conventional hydrogel sheet, which is prepared individually in alignment with an array of electrodes, the hydrogel sheet may be applied irrespective of placement of electrodes. Also, the hydrogel sheet may have a simple structure, making it easy to produce a large area hydrogel sheet.

The net structure may be configured to limit an ionic conduction in a horizontal direction with respect to a plane of the hydrogel sheet. This allows for transmitting bioelectrical signals in the vertical direction effectively.

The hydrogel sheet may have an adhesive strength to a surface of at least 50 grams per square centimeter (g/cm²). This prevents noise due to poor adhesion of the hydrogel sheet to the human body when the hydrogel sheet transmits a bioelectrical signal. It also avoids skin damage, pain associated with detachment, and cell death in a skin after long-term application of the hydrogel sheet.

A lining may support the hydrogel sheet.

The hydrogel sheet may be configured to allow ionic conduction in a vertical direction with respect to a plane of the hydrogel sheet.

The hydrogel sheet may be prepared by polymerization of a biocompatible polymer.

The biocompatible polymer may be a natural polymer and comprises at least one selected from the group consisting of collagen, gelatin, fibril, alginic acid, hyaluronic acid, chitosan, and dextran.

The biocompatible polymer may be a synthetic polymer and comprises at least one selected from the group consisting of polyethyleneglycol, poly2-hydroxyethylmethacrylate (PHEMA), poly(N,N-ethylaminoethyl methacrylate), polyacrylic acid (PAAc), polylactide (PLC), polyglycolide (PGA), polycaprolactone (PCL), poly(caprolactonelactide) random copolymer (PCLA), poly(glycolide-co-ε-caprolactone) random copolymer (PCGA), poly(lactic-co-glycolic acid) random copolymer (PLGA), and polyacrylamide.

In another general aspect, there is provided a sensor platform incluiding a hydrogel sheet comprising a plurality of conductive particles and an electrolyte; and a plurality of electrodes disposed on the hydrogel sheet, wherein the conductive particles are arranged in a vertical direction with respect to a plane of the hydrogel sheet.

The hydrogel sheet may be flexible. This avoids inconvenience when attached to the human body.

The hydrogel sheet may have an anisotropic ionic conductivity. Even when a plurality of electrodes are disposed at arbitrary locations in a disordered arrangement on the hydrogel sheet, bioelectrical signals may be measured at a plurality of regions on a human body without causing an electrical interference between the electrodes. Unlike a conventional hydrogel sheet, which is prepared individually in alignment with an array of electrodes, the hydrogel sheet may be applied irrespective of placement of electrodes. Also, the hydrogel sheet may have a simple structure, making it easy to produce a large area hydrogel sheet.

The anisotropic ionic conductivity may represent an impedance less than or equal to 2 kiloohms (kohm) at 10 hertz (Hz) in the vertical direction with respect to the plane of the hydrogel sheet, and an impedance greater than or equal to 10 kohms at 10 Hz in a horizontal direction with respect to the plane of the hydrogel sheet, when the impedance is measured between the electrodes at a spacing greater than or equal to 5 centimeters (cm).

The conductive particles may be arranged to allow an ionic conduction in the vertical direction with respect to the plane of the hydrogel sheet. This allows for transmitting bioelectrical signals in the vertical direction effectively.

The conductive particles may include a non-polarizable metal.

The conductive particles may include a metal and an insoluble metallic salt of the metal.

The conductive particles may include a non-polarizable metal and an oxide or insoluble metallic salt of the metal.

The conductive particles may include a core-shell particle, and the core comprises the non-polarizable metal, and the shell comprises the oxide or insoluble metallic salt of the metal.

The conductive particles may include a magnetic metal.

A lining may support the hydrogel sheet.

The conductive particles may include a valuable metal.

The conductive particles may have a diameter between 1 nanometer (nm) and 1,000 micrometer (µm).

The conductive particles may have a diameter between 2 nm and 100 µm; and the conductive particles may include at least one of a metallic material, a magnetic material, or a magnetic alloy.

In another general aspect, there is provided a method of preparing a sensor platform, the method including disposing a net structure on a lining; forming a hydrogel sheet including the net structure by applying a mixed solution including an electrolyte onto the net structure; curing the hydrogel sheet; and forming a plurality of electrodes on the hydrogel sheet.

The mixed solution including the electrolyte may include a biocompatible electrolyte, a monomer, a cross-linker, and a photoinitiator.

The mixed solution may include a chain extender.

In another general aspect, there is provided a method of preparing a sensor platform, the method including mixing a mixed solution including an electrolyte with a conductive particle to prepare a mixed solution including the conductive particle; forming a hydrogel sheet by applying the mixed solution including the conductive particle onto a lining; arranging the conductive particle in a vertical direction with respect to a plane of the hydrogel sheet; curing the applied mixed solution; and forming a plurality of electrodes on the hydrogel sheet.

The conductive particle includes a magnetic metal, and the arranging of the conductive particle in the vertical direction comprises arranging the conductive particle in the vertical direction with respect to the plane of the hydrogel sheet using magnetic properties.

The mixed solution including the electrolyte may include a biocompatible electrolyte, a monomer, a cross-linker, and a photoinitiator.

Other features and aspects will be apparent from the following detailed description, the drawings, and the claims.

### BRIEF DESCRIPTION OF THE DRAWINGS

FIG. 1 is a diagram illustrating an example of a sensor platform.
FIGS. 2A and 2B are diagrams illustrating examples of sensor platforms having a plurality of electrodes disposed at different locations.
FIG. 3 is a diagram illustrating an example of a sensor platform.
FIG. 4 is a diagram illustrating an example of a method of preparing a sensor platform.
FIG. 5 is a diagram illustrating an example of a method of preparing a sensor platform.
FIG. 6 is a diagram illustrating an example of use of a sensor platform in a biosignal measurement system.
FIG. 7 is a diagram illustrating an example of impedance between three electrodes of a biosignal measurement device.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

The following detailed description is provided to assist the reader in gaining a comprehensive understanding of the methods, apparatuses, and/or systems described herein. Accordingly, various changes, modifications, and equivalents of the methods, apparatuses, and/or systems described herein will be suggested to those of ordinary skill in the art. Also, description of well-known functions and constructions may be omitted for increased clarity and conciseness.

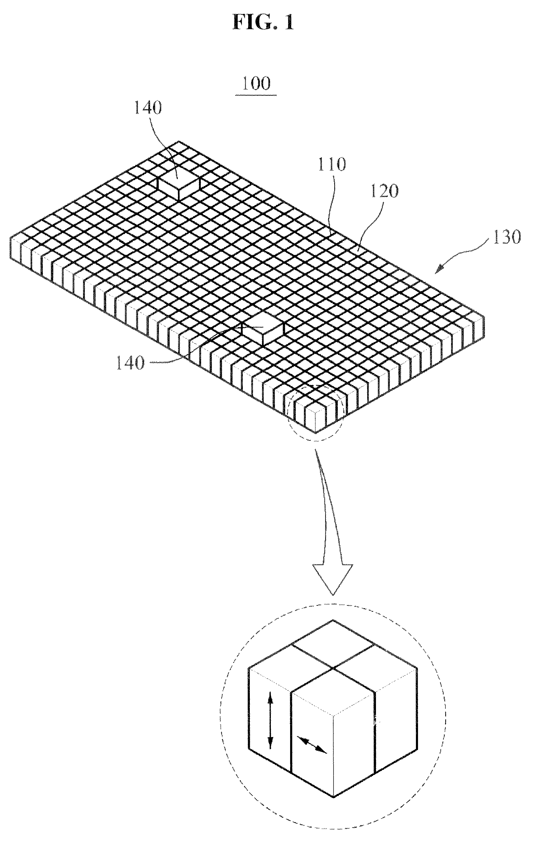

FIG. 1 is a diagram illustrating an example of a sensor platform 100. Referring to FIG. 1, the sensor platform 100 may include a net structure 110, a hydrogel sheet 130 including an electrolyte 120, and a plurality of electrodes 140. The net structure 110 may support the electrolyte 120, and may include a material having an insulating property. The net structure 110 may have a shape that allows ions to travel in a vertical direction with respect to the plane of the hydrogel sheet 130 and to disallow ions to travel in a horizontal direction with respect to the plane of the hydrogel sheet 130. As shown in FIG. 1, the net structure 110 may be in the form of a rectangular grid. However, the net structure 110 is not limited to a specific shape, and may include any other shape, such as, for example, a triangular shape, a pentagonal shape, a hexagonal shape, and a circular shape. The net structure 110 may be made from a member, a fabric, or a mesh having a desired shape. For example, the net structure 110 may be prepared by printing a micro-cell pattern of a wax or an elastomer on a non-woven fabric, followed by transcription using photopolymerization. Due to a shape of the net structure 110, a conductive path may be formed in the vertical direction and electrical isolation may be provided in the horizontal direction to prevent ions from migrating in the horizontal direction with respect to the plane of the hydrogel sheet 130. The net structure 110 may have a non-ionic conductivity to limit an ionic conduction in the horizontal direction with respect to the plane of the hydrogel sheet 130, however, the net structure 110 is not limited in this regard.

The hydrogel sheet 130 may contain moisture and electrolyte 120, and the hydrogel sheet 130 may be prepared by polymerization of a polymer of high biocompatibility. A hydrophilic polymer, , which is used to prepare the hydrogel sheet 130 may be a natural polymer and may include, but is not limited to, at least one selected from the group consisting of collagen, gelatin, fibril, alginic acid, hyaluronic acid, chitosan, or dextran. A hydrophilic polymer may also be a synthetic polymer and may include, but is not limited to, at least one selected from the group consisting of polyethyleneglycol, poly2-hydroxyethylmethacrylate (PHEMA), poly(N,N-ethylaminoethyl methacrylate), polyacrylic acid (PAAc), polylactide (PLC), polyglycolide (PGA), polycaprolactone (PCL), poly(caprolactonelactide) random copolymer (PCLA), poly(glycolide-co-ε-caprolactone) random copolymer (PCGA), poly(lactic-co-glycolic acid) random copolymer (PLGA), or polyacrylamide.

The electrolyte 120, as a biocompatible electrolyte, may include, but is not limited to, at least one selected from the group consisting of potassium chloride (KCI), sodium chloride (NaCl), sodium sulfate (Na₂SO₄), lithium perchlorate (LiClO₄), potassium sulfate (K₂SO₄), lithium chloride (LiCl), potassium nitrate (KNO₃), sodium nitrate (NaNO₃), lithium sulfate (Li₂SO₄), lithium nitrate (LiNO₃), sodium perchlorate (NaClO₄), or potassium perchlorate (KClO₄). The plurality of electrodes 140 may include, but is not limited to, at least one selected from the group consisting of a metal, a conductive metallic oxide, or a conductive polymer.

The hydrogel sheet 130 may have an anisotropic ionic conductivity. To transmit bioelectrical signals in the vertical direction effectively, the anisotropic ionic conductivity may be an ability to move ions in the vertical direction of the hydrogel sheet 130, while blocking ion movement in the horizontal direction of the hydrogel sheet 130.

The anisotropic ionic conductivity may represent impedance less than or equal to 2 kiloohms (kohm) at 10 hertz (Hz) in the vertical direction with respect to the plane of the hydrogel sheet 130 and impedance greater or equal to 10 kohms at 10 Hz in the horizontal direction with respect to the plane of the hydrogel sheet 130, when measured between electrodes arranged at a spacing greater than or equal to 5 centimeters (cm), however, the anisotropic ionic conductivity is not limited in this regard. Impedance in a particular direction may be a factor for determining an ionic conductivity. When impedance in the vertical direction with respect to the plane of the hydrogel sheet 130 is greater than 2 kohms at 10 Hz, a sensitivity for bio-potential measurement may be reduced due to high impedance. The Association for the Advancement of Medical Instrumentation (AAMI) recommends that impedance between two bioelectrodes for electrocardiogram (ECG) measurement be less than or equal to 2 kohms at 10 Hz when the bioelectrodes are attached such that they face one another. When impedance in the horizontal direction with respect to the plane of the hydrogel sheet 130 is less than 10 kohms at 10 Hz, an electrical shunt may be generated between the two points intended to measure a bio-potential, and may consequently produce an error in the bio-potential measurement.

Accordingly, a high ionic conductivity in the vertical direction with respect to the plane of the hydrogel sheet 130 and a low ionic conductivity in the horizontal direction with respect to the plane of the hydrogel sheet 130 may be achieved. The hydrogel sheet 130 may be attached to a bioelectrical signal measurement device without conducting a special alignment between the hydrogel sheet 130 and the device, to provide an electrical access over various regions of the human body.

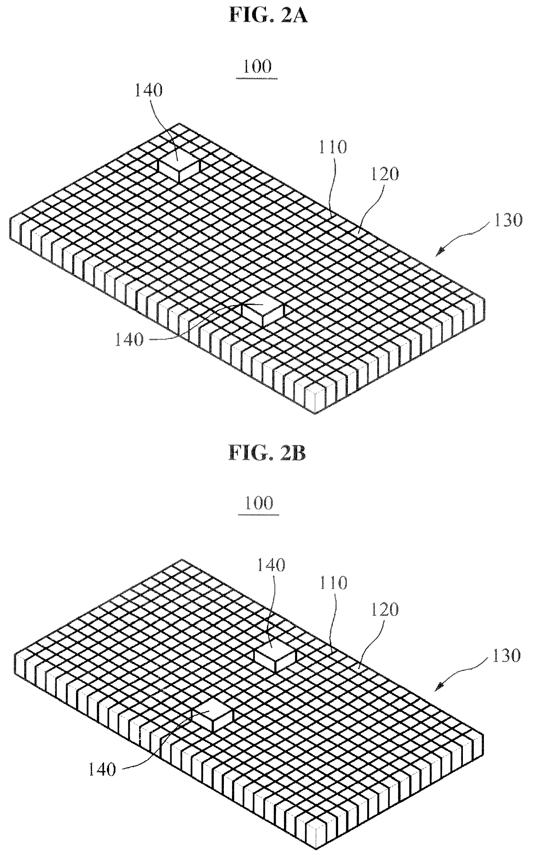

FIGS. 2A and 2B are diagrams illustrating examples of sensor platforms having a plurality of electrodes disposed at different locations. Although the non-exhaustive examples shown in FIGS 2A and 2B show two electrodes on the hydrogel sheet 130, a plurality of electrodes may be used without departing from scope of the illustrative examples described.

As shown in FIGS. 2A and 2B, an ionic conductivity is high, at each location, in the vertical direction and is low in the horizontal direction with respect to the plane of the hydrogel sheet 130. Thus, even when the plurality of electrodes 140 are disposed at arbitrary locations in a disordered arrangement on the hydrogel sheet 130, bioelectrical signals may be measured at a plurality of regions on a human body without causing an electrical interference between the electrodes 140. Unlike a conventional hydrogel sheet, which is prepared individually in alignment with an array of electrodes, the hydrogel sheet 130 may be applied irrespective of placement of electrodes. Also, the hydrogel sheet 130 may have a simple structure, making it easy to produce a large area hydrogel sheet. The hydrogel sheet 130 may also be tailored to an appropriate size and may be applicable irrespective of a type of a bioelectrical signal measurement system.

The hydrogel sheet 130 may be flexible so as not to cause inconvenience when attached to the human body, however, other types of hydrogel sheets may be used without departing from scope of the illustrative examples described.

The hydrogel sheet 130 may have an adhesive strength to prevent noise due to poor adhesion of the hydrogel sheet 130 to the human body when the hydrogel sheet 130 transmits a bioelectrical signal. Also, the adhesive strength of the hydrogel sheet 130 may be such that it avoids skin damage, pain associated with detachment, and cell death in a skin after long-term application of the hydrogel sheet 130. The hydrogel sheet 130 may have an adhesive strength to the skin greater than or equal to a predetermined level, which does not require the use of a separate adhesive. The adhesive strength of the hydrogel sheet 130 to the skin may be greater than or equal to about 50 grams per square centimeter (g/cm²), however, other levels of adhesive strength of the hydrogel sheet 130 to the skin may be used without departing from scope of the illustrative examples described.

The sensor platform 100 may further include a lining to support the hydrogel sheet 130. However, the sensor platform 100 may be used without a lining without departing from scope of the illustrative examples described. The lining may protect a surface of the hydrogel sheet 130, and may be removed before attaching the hydrogel sheet 130 to the human body.

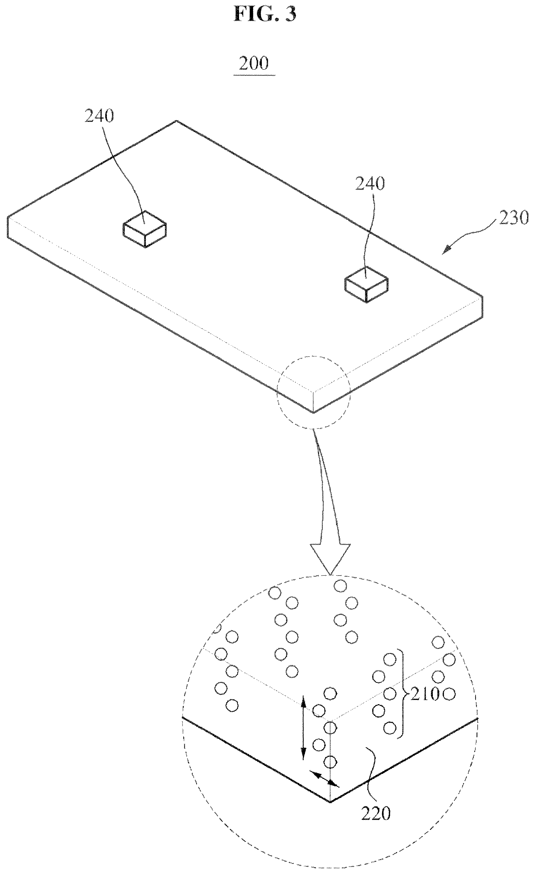

FIG. 3 is a diagram illustrating an example of a sensor platform 200. Referring to FIG. 3, the sensor platform 200 may include conductive particles 210, a hydrogel sheet 230 including an electrolyte 220, and a plurality of electrodes 240.

The conductive particles 210 may allow an ionic conduction in a vertical direction with respect to a plane of the hydrogel sheet 230, however, the illustrative examples described are not limited in this regard. The conductive particles 210 may have an anisotropic ionic conductivity due to a high-density arrangement in the vertical direction with respect to the plane of the hydrogel sheet 230.

The conductive particles 210 may include, but are not limited to, a non-polarizable metal. The non-polarizable metal may include, but is not limited to, metals in Group 1, metals in Group 2, metals in Group 3, mixtures of two or more of these metals, and alloys of one or more of these metals with carbon, silicon, boron, and other metals. The conductive particles 210 may also include, but are not limited to, a valuable metal such as, for example, silver/silver chloride (Ag/AgCl) or gold (Au), stainless steel, and tungsten. The conductive particles 210 may also include, but are not limited to, a metal and an insoluble metallic salt of the metal. The conductive particles 210 may also include, but are not limited to, a non-polarizable metal and an oxide or insoluble metallic salt of the metal. The conductive particles 210 may also correspond to a core-shell particle, where the core may include a non-polarizable metal and the shell may include an oxide or insoluble metallic salt of the metal. The conductive particles 210 may also include, but are not limited to, a magnetic metal. When the conductive particles 210 includes a magnetic metal, vertical arrangement may be enabled, *i.e.,* the conductive particles 210 may allow an ionic conduction in a vertical direction with respect to a plane of the hydrogel sheet 230.

The conductive particles 210 are not limited to a particular particle as long as the particle has a diameter in a range of about 1 nanometer (nm) to 1,000 micrometer (µm). In another non-exhaustive example, the diameter of the conductive particles 210 may be about 2 nm to 100 µm, and may correspond to, for example, a particle of a metallic material, a magnetic material, or a magnetic alloy. The metallic material may include, but is not limited to, at least one selected from the group consisting of platinum (Pt), palladium (Pd), Ag, copper (Cu), and Au.

The magnetic material may include, but is not limited to, at least one selected from the group consisting of cobalt (Co), iron (Fe), nickel (Ni), manganese (Mn), gadolinium (Gd), molybdenum (Mo), MM'₂O₄, and MₓO_{y} in which each of M and M' denotes, independently, Co, Fe, Ni, Mn, zinc (Zn), Gd, or Cr, 0 < x ≤ 3, and 0 < y ≤ 5.

The magnetic alloy may include, but is not limited to, at least one selected from the group consisting of cobalt copper (CoCu), cobalt platinum (CoPt), iron platinum (FePt), cobalt samarium (CoSm), nickel iron (NiFe), or nickel iron cobalt (NiFeCo).

The hydrogel sheet 230 and the electrolyte 220 may correspond to the hydrogel sheet 130 and the electrolyte 120, respectively. The description of hydrogel sheet 130 and the electrolyte 120 is also applicable to the hydrogel sheet 230 and the electrolyte 220, respectively, and thus will not be repeated here.

Similar to the sensor platform including the net structure, the sensor platform 230 including the conductive particles 210 may enable measurement of a bioelectrical signal at a plurality of locations on a human body without causing an electrical interference between electrodes. Unlike a conventional hydrogel sheet, which is prepared individually in alignment with an array of electrodes, the hydrogel sheet 230 may be applied irrespective of placement of electrodes. Also, the hydrogel sheet 230 may have a simple structure, making it easy to produce a large area hydrogel sheet. The hydrogel sheet 230 may also be tailored to an appropriate size and may be applicable irrespective of a type of the bioelectrical signal measurement system.

The hydrogel sheet 230 may be so flexible not to cause inconvenience when attached to the human body, however, other types of hydrogel sheets may be used without departing from scope of the illustrative examples described.

The hydrogel sheet 230 may have an anisotropic ionic conductivity and adhesive strength similar to that of hydrogel sheet 130. The anisotropic ionic conductivity and adhesive strength of hydrogel sheet 130 are described above, and thus will not be repeated here. The sensor platform 200 may further include a lining to support the hydrogel sheet 230. The lining for sensor platform 200 may be similar to the lining for sensor platform 100, which is described above, and thus will not be repeated here.

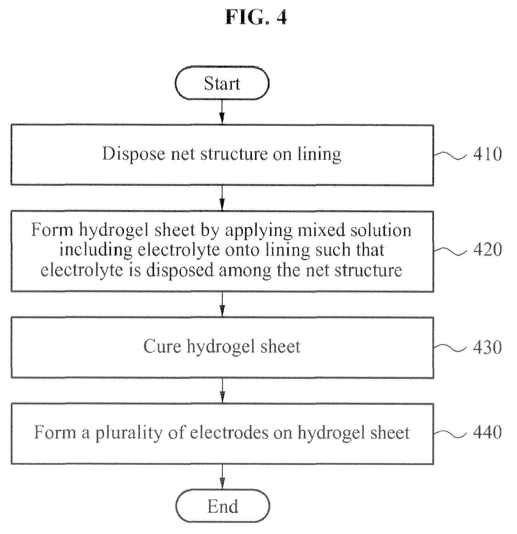

FIG. 4 is a diagram illustrating an example of a method of preparing a sensor platform. The operations in FIG. 4 may be performed in the sequence and manner as shown, although the order of some operations may be changed or some of the operations omitted without departing from scope of the illustrative examples described. Many of the operations shown in FIG. 4 may be performed in parallel or concurrently. The description of FIGS. 1-3 is also applicable to FIG. 4, and thus will not be repeated here.

Referring to FIG. 4, in 410, a net structure may be disposed on a lining. The net structure may be disposed on the lining to support the hydrogel sheet on the lining and to protect a surface of the hydrogel sheet.

In 420, a hydrogel sheet including the net structure may be formed by applying a mixed solution including an electrolyte onto the lining such that the electrolyte is disposed among the net structure. The mixed solution including the electrolyte may include a biocompatible electrolyte, a monomer, a cross-linker, and a photoinitiator. In a non-exhaustive example, the biocompatible electrolyte, the monomer, the cross-linker, and the photoinitiator may be added to the mixed solution before introducing the mixed solution onto the lining.

The cross-linker may be used to prepare a hydrogel sheet having appropriate mechanical properties, such as, for example, a desired tensile strength. The cross-linker may include, but is not limited to, ethylene glycol dimethyl acrylate, triethanolamine (TEOA), a compound having an aldehyde group at a terminal, for example, polyaldehydes such as glutaraldehyde, dialdehyde starch, and succinaldehyde.

The photoinitiator may be used to induce the photopolymerization of the monomer, and may include, but is not limited to, 2,2-dimethoxy-2-phenylacetophenone (DMPA), 2-hydroxy-2-methylpropiophenone (HOMPP), and Irgacure 2959.

The mixed solution including the electrolyte may further include a chain extender, and the chain extender may include, but is not limited to, hexamethylenediamine, m-phenylenediamine, and combinations thereof.

In 430, the hydrogel sheet may be cured. The curing may be carried out using thermal cure or ultraviolet (UV) cure.

In 440, the plurality of electrodes may be formed on the hydrogel sheet. The plurality of electrodes may be disposed at an arbitrary location, rather than at a uniform spacing, to acquire a bioelectrical signal stably irrespective of placement of the electrodes.

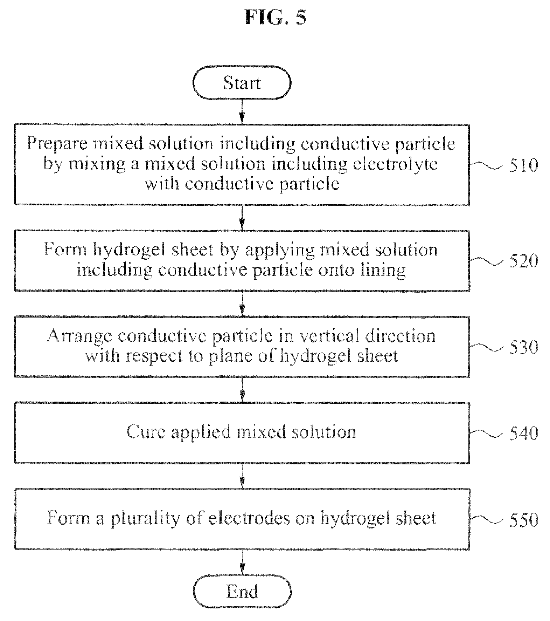

FIG. 5 is a diagram illustrating an example of a method of preparing a sensor platform. The operations in FIG. 5 may be performed in the sequence and manner as shown, although the order of some operations may be changed or some of the operations omitted without departing from scope of the illustrative examples described. Many of the operations shown in FIG. 5 may be performed in parallel or concurrently. The description of FIGS. 1-4 is also applicable to FIG. 5, and thus will not be repeated here.

Referring to FIG. 5, in 510, a mixed solution including an electrolyte with a conductive particles may be prepared by mixing the mixed solution including the electrolyte with the conductive particles.

The mixed solution including the electrolyte may include, but is not limited to, a biocompatible electrolyte, a monomer, a cross-linker, and a photoinitiator.

In 520, the hydrogel sheet may be formed by applying the mixed solution including the conductive particles onto the lining.

In 530, the conductive particles may be arranged in the vertical direction with respect to the plane of the hydrogel sheet. As a non-exhaustive example, when the conductive particles includes a magnetic metal, the conductive particles may be arranged in the vertical direction with respect to the plane of the hydrogel sheet in the presence of a magnetic field formed using magnetic properties.

In 540, the applied mixed solution may be cured. The curing may be carried out using thermal cure or UV cure.

In 550, a plurality of electrodes may be formed on the hydrogel sheet. The plurality of electrodes may be disposed at an arbitrary location, rather than at a uniform spacing, to acquire a bioelectrical signal stably irrespective of placement of the electrodes.

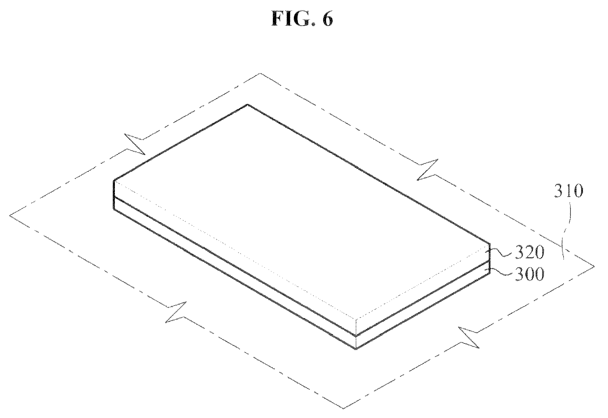

FIG. 6 is a diagram illustrating an example of use of a sensor platform in a biosignal measurement system. Referring to FIG. 6, the biosignal measurement system may be formed by disposing a sensor platform 300 on a skin 310 and by coupling a biosignal measurement device 320 onto the sensor platform 300. The sensor platform 300 may correspond to a sensor platform including a net structure according to a non-exhaustive example, and a sensor platform including a conductive particles according to another non-exhaustive example. The following non-exhaustive examples are only provided for illustrative purposes only and do not limit the scope of the disclosure in any way.

In a non-exhaustive example, a net structure is placed on a lining, and a mixed solution including a monomer, a cross-linker, a photoinitiator, an electrolyte, a moisturizer, and water is applied onto the net structure. A hydrogel sheet including the net structure is formed after removing the excess mixed solution. The excess mixed solution is removed by placing another lining on the mixed solution and rolling a roller over the applied mixed solution. The prepared hydrogel sheet is placed in a UV curer and cured for fifteen minutes.

A biosignal measurement device is prepared by contacting a flexible printed circuit board (FPCB) having three electrode patterns with the prepared hydrogel sheet at both sides. Using the prepared biosignal measurement device, impedance between the electrodes is measured.

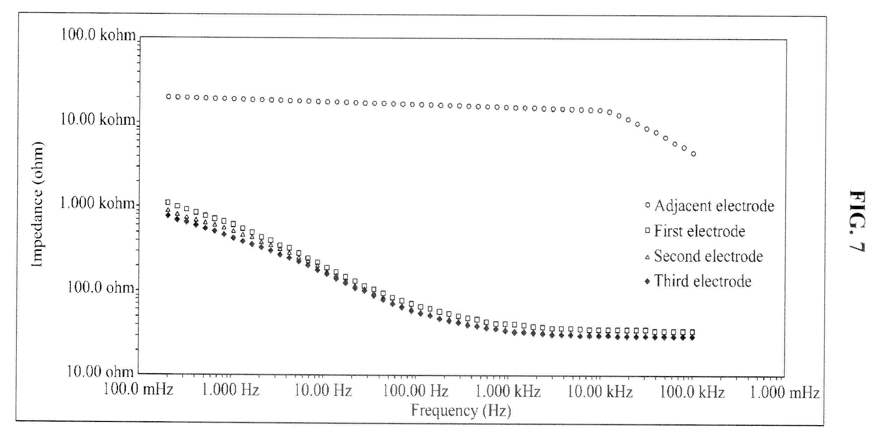

FIG. 7 is a diagram illustrating an example of impedance properties of a biosignal measurement device with three electrodes arranged at a spacing of 5 cm. When an electrode for testing was adhered to an opposite surface of the hydrogel sheet for each electrode of the sensor platform, it was found that impedance between each electrode pair was 200 ohms at 10 Hz sufficiently lower than AAMI standard of 2 kohms. Also, it was found that impedance between adjacent electrodes on the same horizontal plane included in the sensor platform was 20 times or more higher at 1 Hz and 100 times or more higher at 10 Hz than impedance between electrodes facing one another. It was found from this impedance difference that the hydrogel sheet had an anisotropic ionic conductivity.

In another non-exhaustive example, a mixed solution including conductive particles is prepared by mixing a mixed solution including a monomer, a cross-linker, a photoinitiator, an electrolyte, a moisturizer, and water, with particles in which silver chloride is formed on a surface of a silver-plated nickel particles. The mixed solution including the conductive particles is applied on a lining. A hydrogel sheet is formed after removing an excess mixed solution. The excess mixed solution is removed by rolling a roller over the applied mixed solution with another lining put on the mixed solution. The particles in which silver chloride are formed on the surface of the silver-plated nickel particles are arranged in a vertical direction with respect to a plane of the hydrogel sheet. The hydrogel sheet is placed in a UV curer and cured for fifteen minutes.

Impedance of the biosignal measurement device formed from the hydrogel sheet is measured. Impedance between electrodes facing one another was 220 ohms at 10 Hz, sufficiently lower than the AAMI standard of 2 kohms. Also, it was found that impedance between adjacent electrodes on the same horizontal plane was 18 times or more higher at 1 Hz and 120 times or more higher at 10 Hz than impedance between electrodes facing one another. It was also found from this impedance difference that the hydrogel sheet including the conductive particles had an anisotropic ionic conductivity of an effective level.

A number of examples have been described above. Nevertheless, it should be understood that various modifications may be made. For example, suitable results may be achieved if the described techniques are performed in a different order and/or if components in a described system, architecture, device, or circuit are combined in a different manner and/or replaced or supplemented by other components or their equivalents. The invention is defined by the attached claims 1-14.

## Claims

1. A sensor platform (100; 200) comprising:
a hydrogel sheet (130; 230) comprising a plurality of conductive particles (210) arranged in a vertical direction with respect to a plane of the hydrogel sheet (230) and an electrolyte (220); and
a plurality of electrodes (140; 240) disposed on the hydrogel sheet (130; 230).

2. The sensor platform (100; 200) of claim 1, wherein the hydrogel sheet (130; 230) has an anisotropic ionic conductivity.

3. The sensor platform (100; 200) any one of claims 1 or 2, wherein the hydrogel sheet (130; 230) has an adhesive strength to a surface of at least 50 grams per square centimeter (g/cm²).

4. The sensor platform (100; 200) of any one of claims 1 to 3, further comprising a lining to support the hydrogel sheet (130; 230).

5. The sensor platform (100; 200) of any one of claims 1 to 4, wherein the hydrogel sheet (130; 230) is prepared by polymerization of a biocompatible polymer.

6. The sensor platform (100; 200) of claim 5, wherein the biocompatible polymer is a natural polymer and comprises at least one selected from the group consisting of collagen, gelatin, fibril, alginic acid, hyaluronic acid, chitosan, and dextran; and/or
wherein the biocompatible polymer is a synthetic polymer and comprises at least one selected from the group consisting of polyethyleneglycol, poly2-hydroxyethylmethacrylate (PHEMA), poly(N,N-ethylaminoethyl methacrylate), polyacrylic acid (PAAc), polylactide (PLC), polyglycolide (PGA), polycaprolactone (PCL), poly(caprolactonelactide) random copolymer (PCLA), poly(glycolide-co-ε-caprolactone) random copolymer (PCGA), poly(lactic-co-glycolic acid) random copolymer (PLGA), and polyacrylamide.

7. The sensor platform (100; 200) of any one of claims 3 to 6, wherein the hydrogel sheet (130; 230) has an anisotropic ionic conductivity and wherein the anisotropic ionic conductivity represents an impedance less than or equal to 2 kiloohms (kohm) at 10 hertz (Hz) in the vertical direction with respect to the plane of the hydrogel sheet (130; 230), and an impedance greater than or equal to 10 kohms at 10 Hz in a horizontal direction with respect to the plane of the hydrogel sheet (130; 230), when the impedance is measured between the electrodes (140; 240) at a spacing greater than or equal to 5 centimeters (cm).

8. The sensor platform (100; 200) of any one of claims 1 to 7, wherein the conductive particles (210) are arranged to allow an ionic conduction in the vertical direction with respect to the plane of the hydrogel sheet (130; 230).

9. The sensor platform (100; 200) of any one of claims 1 to 8, wherein the conductive particles (210) comprise
a non-polarizable metal, and/or
a metal and an insoluble metallic salt of the metal, and/or
a non-polarizable metal and an oxide or insoluble metallic salt of the metal, in particular a core-shell particle, the core comprising the non-polarizable metal, and the shell comprising the oxide or insoluble metallic salt of the metal, and/or
a magnetic metal, and/or
a valuable metal.

10. The sensor platform (100; 200) of claims 1 to 9, wherein the conductive particles (210) have a diameter between 1 nanometer (nm) and 1,000 micrometer (µm).

11. The sensor platform (100; 200) of claims 1 to 10, wherein:
the conductive particles (210) have a diameter between 2 nm and 100 µm; and
the conductive particles (210) comprise at least one of a metallic material, a magnetic material, or a magnetic alloy.

12. A method of preparing a sensor platform (100; 200), the method comprising:
mixing a mixed solution including an electrolyte (220) with a conductive particle to prepare a mixed solution including the conductive particle (210),
forming a hydrogel sheet (130; 230) by applying the mixed solution including the conductive particle (210) onto a lining, arranging the conductive particle (210) in a vertical direction with respect to a plane of the hydrogel sheet (130; 230);
curing the applied mixed solution; and
forming a plurality of electrodes (140; 240) on the hydrogel sheet (130; 230).

13. The method of claim 12, wherein the mixed solution including the electrolyte comprises a biocompatible electrolyte, a monomer, a cross-linker, and a photoinitiator and/or a chain extender.

14. The method of claims 12 or 13, wherein the conductive particle (210) comprises a magnetic metal, and the arranging of the conductive particle (210) in the vertical direction comprises arranging the conductive particle (210) in the vertical direction with respect to the plane of the hydrogel sheet (130; 230) using magnetic properties.

## Patentansprüche

1. Sensorplattform (100; 200), umfassend:
eine Hydrogelfolie (130; 230), die eine Vielzahl leitfähiger Partikel (210) umfasst, die in einer vertikalen Richtung in Bezug auf eine Ebene der Hydrogelfolie (230) angeordnet sind, und ein Elektrolyt (220);
und eine Vielzahl von Elektroden (140; 240), die auf der Hydrogelfolie (130; 230) angeordnet sind.

2. Sensorplattform (100; 200) nach Anspruch 1, bei der die Hydrogelfolie (130; 230) eine anisotrope ionische Leitfähigkeit hat.

3. Sensorplattform (100; 200) nach einem der Ansprüche 1 oder 2, bei der die Hydrogelfolie (130; 230) eine Haftfestigkeit an einer Oberfläche von wenigstens 50 Gramm pro Quadratzentimeter (g/cm²) hat.

4. Sensorplattform (100; 200) nach einem der Ansprüche 1 bis 3, weiterhin umfassend eine Kaschierung, um die Hydrogelfolie (130; 230) zu stützen.

5. Sensorplattform (100; 200) nach einem der Ansprüche 1 bis 4, bei der die Hydrogelfolie (130; 230) durch Polymerisation eines biokompatiblen Polymers vorbereitet wird.

6. Sensorplattform (100; 200) nach Anspruch 5, bei der das biokompatible Polymer ein natürliches Polymer ist und wenigstens ein Element aus der Gruppe umfasst, die besteht aus Kollagen, Gelatin, Fibril, Alginsäure, Hyaluronsäure, Chitosan und Dextran; und/oder
bei der das biokompatible Polymer ein synthetisches Polymer ist und wenigstens ein Element umfasst, das aus der Gruppe gewählt ist, die besteht aus Polyethylenglycol, Poly2-Hydroxyethylmethacrylat (PHEMA), Poly(N, N-Ethylaminoethyl Methacrylat), Polyacrylsäure (PAAc), Polylactid (PLC), Polyglycolid (PGA), Polycaprolacton (PCL), Poly(Caprolactonelactid) Random-Copolymer (PCLA), Poly(glycolid-co-ε-Caprolacton) Random-Copolymer (PCGA), Poly(lactid-co-Glycolid-Säure) Random-Copolymer (PLGA) und Polyacrylamid.

7. Sensorplattform (100; 200) nach einem der Ansprüche 3 bis 6, bei der die Hydrogelfolie (130; 230) eine anisotrope ionische Leitfähigkeit hat und die anisotrope ionische Leitfähigkeit eine Impedanz repräsentiert, die kleiner oder gleich 2 Kiloohm (kOhm) bei 10 Hertz (Hz) in der vertikalen Richtung in Bezug auf die Ebene der Hydrogelfolie (130; 230) ist, und eine Impedanz, die größer oder gleich 10 kOhm bei 10 Hz in einer horizontalen Richtung in Bezug auf die Ebene der Hydrogelfolie (130; 230) ist, wenn die Impedanz zwischen den Elektroden (140; 240) in einem Abstand gemessen wird, der größer oder gleich 5 Zentimeter (cm) ist.

8. Sensorplattform (100; 200) nach einem der Ansprüche 1 bis 7, bei der die leitfähigen Partikel (210) derart angeordnet sind, dass sie eine ionische Leitung in der vertikalen Richtung in Bezug auf die Ebene der Hydrogelfolie (130; 230) gestattet.

9. Sensorplattform (100; 200) nach einem der Ansprüche 1 bis 8, bei der die leitfähigen Partikel (210) umfassen:
ein nicht polarisierbares Metall und/oder
ein Metall und ein nicht lösliches metallisches Salz des Metalls und/oder
ein nicht polarisierbares Metall und ein Oxid oder nicht lösliches metallisches Salz des Metalls, insbesondere einen Kern-Hüllen-Partikel, wobei der Kern das nicht polarisierbare Metall umfasst und die Hülle das Oxid oder das nicht lösliche metallische Salz des Metalls umfasst, und/oder
magnetisches Metall und/oder
ein Edelmetall.

10. Sensorplattform (100; 200) nach Anspruch 1 bis 9, bei der die leitfähigen Partikel (210) einen Durchmesser zwischen 1 Nanometer (nm) und 1.000 Mikrometer (µm) haben.

11. Sensorplattform (100; 200) nach Anspruch 1 bis 10, bei der
die leitfähigen Partikel (210) einen Durchmesser zwischen 2 nm und 100 µm haben; und
die leitfähigen Partikel (210) ein metallisches Material, ein magnetisches Material oder eine magnetische Legierung umfassen.

12. Verfahren für die Vorbereitung einer Sensorplattform (100; 200), wobei das Verfahren umfasst:
Mischen einer gemischten Lösung, die ein Elektrolyt (220) umfasst, mit einem leitfähigen Partikel, um eine gemischte Lösung vorzubereiten, die den leitfähigen Partikel (210) enthält,
Ausbilden einer Hydrogelfolie (130; 230) durch Aufbringen der gemischten Lösung, die den leitfähigen Partikel (210) umfasst, auf eine Kaschierung, anordnen des leitfähigen Partikels (210) in einer vertikalen Richtung in Bezug auf eine Ebene der Hydrogelfolie (130; 230);
Aushärten der aufgebrachten gemischten Lösung; und
Ausbilden einer Vielzahl von Elektroden (140; 240) auf der Hydrogelfolie (130; 230).

13. Verfahren nach Anspruch 12, bei dem die gemischte Lösung, die das Elektrolyt umfasst ein biokompatibles Elektrolyt, ein Monomer, einen Vernetzer und einen Fotoinitiator und/oder einen Kettenverlängerer umfasst.

14. Verfahren nach Anspruch 12 oder 13, bei dem der leitfähige Partikel (210) ein magnetisches Metall umfasst und das Anordnen des leitfähigen Partikels (210) in der vertikalen Richtung das Anordnen des leitfähigen Partikels (210) in der vertikalen Richtung in Bezug auf die Ebene der Hydrogelfolie (130; 230) unter Verwendung magnetischer Eigenschaften umfasst.

## Revendications

1. Plate-forme de capteurs (100 ; 200) comprenant :
une feuille d'hydrogel (130 ; 230) comprenant une pluralité de particules conductrices (210) agencées en direction verticale par rapport à un plan de la feuille d'hydrogel (230) et un électrolyte (220) ; et
une pluralité d'électrodes (140 ; 240) agencées sur la feuille d'hydrogel (130 ; 230).

2. Plate-forme de capteurs (100 ; 200) selon la revendication 1, dans laquelle la feuille d'hydrogel (130 ; 230) présente une conductivité ionique anisotrope.

3. Plate-forme de capteurs (100 ; 200) selon l'une quelconque des revendications 1 et 2, dans laquelle la feuille d'hydrogel (130 ; 230) présente une force d'adhésion à une surface d'au moins 50 grammes par centimètre carré (g/cm²).

4. Plate-forme de capteurs (100 ; 200) selon l'une quelconque des revendications 1 à 3, comprenant en outre un revêtement intérieur pour supporter la feuille d'hydrogel (130 ; 230).

5. Plate-forme de capteurs (100 ; 200) selon l'une quelconque des revendications 1 à 4, dans laquelle la feuille d'hydrogel (130 ; 230) est préparée par polymérisation d'un polymère biocompatible.

6. Plate-forme de capteurs (100 ; 200) selon la revendication 5,
dans laquelle le polymère biocompatible est un polymère naturel et comprend au moins un composant sélectionné dans le groupe constitué par le collagène, la gélatine, les fibrilles, l'acide alginique, l'acide hyaluronique, le chitosane et le dextrane ; et/ou
dans laquelle le polymère biocompatible est un polymère synthétique et comprend au moins un composant sélectionné parmi le groupe constitué par le polyéthylène glycol, le poly 2-hydroxyéthyl méthacrylate (PHEMA), le poly (N,N-éthylaminoéthyl méthacrylate), l'acide polyacrylique (PAAc), l'acide polylactique (PLC), le poly(acide glycolique) (PGA), le polycaprolactone (PCL), le copolymère statistique de poly(caprolactone-lactide) (PCLA), le copolymère statistique de poly(glycolide-co-ε-caprolactone) (PCGA), le copolymère statistique de poly(acide lactique-co-glycolique) (PLGA), et le polyacrylamide.

7. Plate-forme de capteurs (100 ; 200) selon l'une quelconque des revendications 3 à 6, dans laquelle la feuille d'hydrogel (130 ; 230) présente une conductivité ionique anisotrope et dans laquelle la conductivité ionique anisotrope représente une impédance inférieure ou égale à 2 kilo-ohms (kΩ) à 10 hertz (Hz) en direction verticale par rapport au plan de la feuille d'hydrogel (130 ; 230), et une impédance supérieure ou égale à 10 kΩ à 10 Hz en direction horizontale par rapport au plan de la feuille d'hydrogel (130 ; 230), quand l'impédance est mesurée entre les électrodes (140 ; 240) avec un espacement supérieur ou égal à 5 centimètres (cm).

8. Plate-forme de capteurs (100 ; 200) selon l'une quelconque des revendications 1 à 7, dans laquelle les particules conductrices (210) sont agencées pour permettre une conduction ionique en direction verticale par rapport au plan de la feuille d'hydrogel (130 ; 230).

9. Plate-forme de capteurs (100 ; 200) selon l'une quelconque des revendications 1 à 8, dans laquelle les particules conductrices (210) comprennent
un métal non polarisable, et/ou
un métal et un sel métallique insoluble du métal, et/ou
un métal non polarisable et un oxyde ou un sel métallique insoluble du métal, en particulier une particule noyau-enveloppe, le noyau comprenant le métal non polarisable, et l'enveloppe l'oxyde ou le sel métallique insoluble du métal, et/ou
un métal magnétique, et/ou
un métal précieux.

10. Plate-forme de capteurs (100 ; 200) selon les revendications 1 à 9, dans laquelle les particules conductrices (210) présentent un diamètre compris entre 1 nanomètre (nm) et 1.000 micromètres (µm).

11. Plate-forme de capteurs (100 ; 200) selon les revendications 1 à 10, dans laquelle :
les particules conductrices (210) présentent un diamètre compris entre 2 nm et 100 µm ; et
les particules conductrices (210) comprennent au moins un matériau parmi un matériau métallique, un matériau magnétique et un alliage magnétique.

12. Procédé de préparation d'une plate-forme de capteurs (100 ; 200), le procédé comprenant :
le mélange d'une solution mélangée incluant un électrolyte (220) avec une particule conductrice pour préparer une solution mélangée incluant la particule conductrice (210),
la formation d'une feuille d'hydrogel (130 ; 230) en appliquant la solution mélangée incluant la particule conductrice (210) sur un revêtement intérieur, en agençant la particule conductrice (210) en direction verticale par rapport à un plan de la feuille d'hydrogel (130 ; 230) ;
le durcissement de la solution mélangée appliquée ; et
la formation d'une pluralité d'électrodes (140 ; 240) sur la feuille d'hydrogel (130 ; 230).

13. Procédé selon la revendication 12, dans lequel la solution mélangée incluant l'électrolyte comprend un électrolyte biocompatible, un monomère, un agent de réticulation, et un photo-initiateur et/ou un allongeur de chaîne.

14. Procédé selon la revendication 12 ou 13, dans lequel la particule conductrice (210) comprend un métal magnétique, et l'agencement de la particule conductrice (210) en direction verticale comprend l'agencement de la particule conductrice (210) en direction verticale par rapport au plan de la feuille d'hydrogel (130 ; 230) en utilisant des propriétés magnétiques.
